# EUROPÄISCHE PATENTANMELDUNG

(11) **EP 1 920 798 A1**
(43) Veröffentlichungstag der Anmeldung: **14.05.2008**
(21) Anmeldenummer: 06023271.7
(22) Anmeldetag: 08.11.2006
(51) Int. Cl.: A61N 5/06

(54) **Infrarotbestrahlungseinrichtung zur Bestrahlung der menschlichen Haut**

(71) Anmelder: Roewer, Norbert, Univ.-Prof. Dr. med., 97082 Würzburg (DE); Broscheit, Jens, Dr. med., 97209 Würzburg (DE)
(72) Erfinder: Roewer, Norbert, Univ.-Prof. Dr. med., 97082 Würzburg (DE); Broscheit, Jens, Dr. med., 97209 Würzburg (DE)
(74) Vertreter: Glawe, Delfs, Moll

(57) **Zusammenfassung**

Gegenstand der Erfindung ist eine Infrarotbestrahlungseinrichtung zur Bestrahlung der menschlichen Haut einer Person, mit einer Infrarotquelle und einer Regeleinrichtung für die Infrarotquelle. Erfindungsgemäß ist vorgesehen, dass zur Leistungsregelung der Infrarotquelle ein Regelkreis vorgesehen ist, in den als Regelgrößen sowohl die Temperatur wenigstens eines Teils der bestrahlten Hautfläche der Person als auch die Kerntemperatur der Person einfließen.

## Beschreibung

Die Erfindung betrifft eine Infrarotbestrahlungseinrichtung zur Bestrahlung der menschlichen Haut einer Person, mit einer Infrarotquelle und einer Leistungsregeleinrichtung für die Infrarotquelle.

In Operationssälen ist zur Gewährleistung optimaler hygienischer Bedingungen ein laminarer Luftstrom von der Decke nach unten notwendig, um zu verhindern dass Keime aufgewirbelt werden. Die notwendige Lufttemperatur beträgt ca. 20°C. Höhere Temperaturen könnten den laminaren Luftstrom abreißen lassen und würden darüber hinaus den arbeiteten Operateur zu sehr belasten.

Die Körperkerntemperatur des Patienten beträgt 37°C und die mittlere Hauttemperatur etwa 32°C. Bei einer Umgebungstemperatur von 20°C und einem laminaren Luftstrom erfolgt daher eine relativ starke Auskühlung des Patienten, der nur durch ein Operationstuch abgedeckt ist. Da unter Narkose die physiologische Wärmeregulation eingeschränkt bzw. ausgeschaltet ist, kann dies zu einer Reihe von Komplikationen führen. Bei den Mechanismen, die zur Auskühlung des Patienten beitragen, handelt es sich um Wärmestrahlung (InfrarotStrahlung, die der Patient aufgrund seiner Temperatur an die Umgebung abstrahlt), Konvektion (vorbeiströmende kalte Luft nimmt einen Teil der Wärme mit), Wärmeleitung (Körperwärme wird hauptsächlich durch die Operationstische abgeleitet) und Verdunstung (Schweiß verdampft und entzieht dem Körper damit Wärme). Darüber hinaus wird Wärme über die Atmung abgegeben, ebenso tritt ein Wärmeverlust aus der Operationswunde auf. Durch den Wärmeverlust besteht am ehesten die Gefahr einer Hypothermie, eine Hypothermie tritt deutlich seltener auf. Die Gefahr einer Hypothermie ist besonders hoch bei Eingriffen mit großen Volumenverschiebungen, lang andauernden Eingriffen (mehr als 2 Stunden), Kindern in den ersten Lebensjahren, Durchführung einer kontrollierten Hypothermie (Kardio- und Neurochirurgie), Schockzuständen (v. a. hämorrhagischer Schock) und Patienten mit Unterkühlung.

Der Patient muss in jedem Fall vor der Narkoseausleitung wiedererwärmt werden, was die Narkosedauer verlängert.

De 195 33 753 C1 offenbart eine Infrarotbestrahlungseinrichtung für eine Person auf einer Liegefläche.

Der Erfindung liegt die Aufgabe zugrunde, eine Einrichtung der eingangs genannten Art zu schaffen, die in der Umgebung eines Operationssaales einfach und sicher handhabbar ist.

Erfindungsgemäß ist vorgesehen, dass zur Leistungsregelung der Infrarotquelle ein Regelkreis vorgesehen ist, in den als Regelgrößen sowohl die Temperatur wenigstens eines Teils der bestrahlten Hautfläche der Person als auch die Kerntemperatur der Person einfließen.

Zunächst seien einige im Rahmen der Erfindung verwendete Begriffe erläutert.

Eine Infrarotbestrahlungseinrichtung ist jegliche Einrichtung, die Infrarotstrahlung (Wellenlänge > 700 nm) gerichtet auf eine Fläche abstrahlen kann. Die Infrarotquelle ist bevorzugt elektrisch betrieben und kann beispielsweise eine Infrarotlampe, ein Infrarotheizstab oder dergleichen sein. Die Leistungsregeleinrichtung erlaubt eine Veränderung der Abstrahlleistung der Einrichtung. Dies kann beispielsweise geschehen durch Veränderung des Abstandes, teilweises Abschirmen der Strahlung oder dergleichen, erfolgt jedoch vorzugsweise durch eine Regelung der Leistung der Infrarotquelle beispielsweise durch Änderung der Versorgungsspannung, durch eine Phasenanschnittsteuerung oder dergleichen.

Zur Leistungsregelung der Infrarotquelle ist ein Regelkreis vorgesehen. In diesen Regelkreis fließen wenigstens zwei Regelgrößen ein. Zum einen ist dies die Temperatur wenigstens eines Teil der bestrahlten Hautfläche der Person. Der Zweck dieser Regelung ist es insbesondere, eine übermäßig starke Bestrahlung der Haut oder eines Teils davon zu verhindern und Folgeschäden wie beispielsweise Verbrennungen zu vermeiden. Die Erfassung der Kerntemperatur der Person als Regelgröße dient zur Prüfung, ob eine ausreichende Wärmezufuhr zur Vermeidung einer Auskühlung stattfindet.

Regelgrößen des Regelkreises sind die Leistungsabgabe der Infrarotquelle und/oder die Größe bzw. Anordnung der bestrahlten Fläche.

In der Umgebung eines Operationssaals ist mit einem Wärmeverlust eines in Narkose befindlichen Patienten von etwa bis zu 160 W zu rechnen. Dies schließt ein die Abstrahlung der Körperoberflächen, die Konvektion von Wärme von der Körperoberfläche an die Umgebungsluft, Wärmeleitung an berührende Flächen (Operationstisch) Wärmeabfuhr durch Verdunstung und Atmung.

Um eine Auskühlung zu vermeiden, muss eine entsprechende Wärmeleistung dem Körper wieder zugeführt werden. Dies kann erfindungsgemäß erfolgen, wobei gemäß einem wesentlichen Merkmal der Erfindung die Überwachung der ausreichenden Wärmezufuhr in erster Linie durch Überwachung der Kerntemperatur des Patienten erfolgt. Da für diese Wärmezufuhr durch Strahlungswärme in der Regel nur ein Bruchteil der Körperoberfläche des Patienten (gesamte Körperoberfläche etwa 1,8 m²) zur Verfügung steht, kommt es darauf an, die Strahlungsleistung auf die bestrahlte Fläche so zu dosieren, dass einerseits die notwendige Wärmezufuhr erreicht wird und andererseits keine Überwärmung der bestrahlten Hautpartien erfolgt. Genau dies leistet der doppelte Regelkreis der Erfindung.

Für die Hauttemperaturmessung können erfindungsgemäß Strahlungssensoren (beispielsweise Pyrometer) und/oder Hautkontakttermometer verwendet werden. Strahlungssensoren haben den Vorteil, dass sie berührungsfrei messen. Sie können beispielsweise an der erfindungsgemäßen Einrichtung angebracht und auf die bestrahlten Hautpartien gerichtet werden.

Hautkontakttermometer können insbesondere dann zur Anwendung kommen, wenn beispielsweise auch mit einem OP-Tuch abgedeckte Hautpartien des Patienten erwärmt werden sollen. Im Rahmen der Erfindung ist es möglich, dass hinreichend infrarotdurchlässige OP-Tücher zur Abdeckung von Teilen des Patienten verwendet werden, durch die dann eine Bestrahlung und entsprechende Erwärmung der Haut erfolgt. Da in einem solchen Fall eine pyrometrische Messung die Temperatur des Tuches und nicht der darunterliegenden Haut messen würde, werden vorteilhafterweise Hautkontakttermometer verwendet.

Zur Messung der Kerntemperatur der Person sind vorzugsweise ebenfalls geeignete Kontakttermometer vorgesehen, die beispielsweise in der Art üblicher Fiebertermometer ausgebildet sein können.

Die Strahlungsleistung der Einrichtung beträgt vorzugsweise 1000 W/m² bestrahlte Hautfläche oder weniger, vorzugsweise 500 W/m² bestrahlter Hautfläche oder weniger. Mit einer Strahlungsleistung von 500 W/m² lassen sich bei der Bestrahlung von Brust und Bauch eines Patienten (Flächenanteil von etwa 18% der Hautoberfläche oder ungefähr 0,32 m²) etwa 160 W Wärmeleistung zuführen. Dies entspricht in etwa dem oben geschilderten Wärmeverlust eines Patienten in Narkose.

Erfindungsgemäß kann vorgesehen sein, dass die primäre Regelgröße des Regelkreises die Kerntemperatur ist. Dies bedeutet, dass die Strahlungsleistung anhand dieser primären Regelgröße so eingestellt wird, dass die gewünschte Kerntemperatur erreicht bzw. gehalten wird.

Die Hauttemperatur kann eine sekundäre Regelgröße sein. Wird eine vorgebbare Hauttemperatur überschritten, bewirkt diese sekundäre Regelgröße eine Leistungsreduzierung oder Veränderung der Strahlungsrichtung, auch wenn die gewünschte Kerntemperatur noch nicht erreicht ist. Auf diese Weise werden Überhitzungserscheinungen der Haut vermieden. Sollte die vorgegebene Kerntemperatur durch Bestrahlung der zur Verfügung stehenden Hautoberfläche nicht erreicht oder gehalten werden können, kann die erfindungsgemäße Einrichtung beispielsweise ein Alarmsignal abgeben.

Die Infrarotstrahlung ist bevorzugt auf einstellbare Flächenbereiche fokussierbar. Dies kann beispielsweise geschehen durch manuelles oder bevorzugt motorisches Verschwenken der gesamten Bestrahlungseinrichtung, durch Verändern der Reflektorstellung, durch Einsatz geeigneter weiterer optischer Fokussierungselemente, durch Einsatz einer Bestrahlungseinrichtung mit einer Mehrzahl von Infrarotquellen, die wahlweise zu- oder abgeschaltet werden oder dergleichen. Auf diese Art und Weise kann die Infrarotstrahlung gezielt auf zu erwärmende Hautbereiche des Patienten gerichtet werden, ohne die Chirurgen bei ihrer Arbeit zu behindern und ohne beispielsweise die Anordnung der Operationsfeldbeleuchtung zu beeinträchtigen.

Im Rahmen der Erfindung kann vorgesehen sein, dass die Einrichtung einen Sensor zur Messung der IR-Reflexivität der bestrahlten Flächen aufweist. Auf diese Art und Weise kann beispielsweise eine automatische Fokussierung auf zu bestrahlende freie Hautflächen erfolgen, da diese eine andere IR-Reflexivität aufweist als beispielsweise Operationstücher. Der Sensor kann in einem Regelkreis mit den Stelleinrichtungen zur Fokussierung der Infrarotstrahlung rückgekoppelt sein und dafür sorgen, dass die Infrarotstrahlung automatisch nur auf Bereiche einer definierten Reflexivität gerichtet wird. Diese Regelung kann dynamisch erfolgen und hat beispielsweise dann besondere Vorteile, wenn ein Chirurg während der Operation in den Strahlungsweg hineingreift. Die Kleidung des Chirurgen wird in aller Regel eine andere IR-Reflexivität als die Haut aufweisen. Bedarfsweise kann auch vorgesehen sein, dass die Kleidung des Chirurgen eine besondere Beschichtung beispielsweise zur Erhöhung der IR-Reflexivität aufweist. Findet während der Operation ein solcher Eingriff in den Strahlungsweg statt, kann die erfindungsgemäße Einrichtung dies automatisch erkennen und die Strahlungsleistung in den entsprechenden Bereichen vermindern bzw. die Infrarotstrahlung neu fokussieren. Es wird so vermieden, dass auch der Chirurg "erwärmt" wird.

Erfindungsgemäß kann dementsprechend vorgesehen sein, dass die Einrichtung eine Umrisserkennung der zu bestrahlenden Hautflächen einer Person durch Messung der IR-Reflexivität der bestrahlten Flächen aufweist. Wenn die Erwärmung durch unmittelbare Bestrahlung der Haut erfolgen soll, wird die entsprechende Einrichtung so eingestellt, dass Wärmestrahlung direkt auf diese Hautpartien gerichtet wird. Wenn die Erwärmung der Haut durch ein IR-durchlässiges Operationstuch erfolgen soll, muss die Einrichtung so eingestellt werden, dass sie die IR-Reflexion dieses Operationstuches erkennt. Es kommen zu diesem Zweck vorzugsweise solche Operationstücher zum Einsatz, deren IR-Reflexivität sich unterscheidet von sonstigen Tüchern und der Kleidung der an der Operation beteiligten Personen.

Bei einer Variante der Erfindung kann die Umrisserkennung der zu bestrahlenden Hautflächen einer Person auch durch Messung der Reflektionen im sichtbaren Bereich (400 bis 700 nm) erfolgen, beispielsweise durch eine der erfindungsgemäßen Einrichtung zugeordnete Kamera. Eine automatische Erkennung der zu bestrahlenden Flächen kann dann beispielsweise anhand der Farbe erfolgen. Beispielsweise kann die Hautfarbe automatisch erkannt werden, alternativ kann vorgesehen sein, dass bei einer Bestrahlung durch IRdurchlässige Operationstücher hindurch diese eine besondere Farbe aufweisen, die ebenfalls von der Kamera erkannt werden kann.

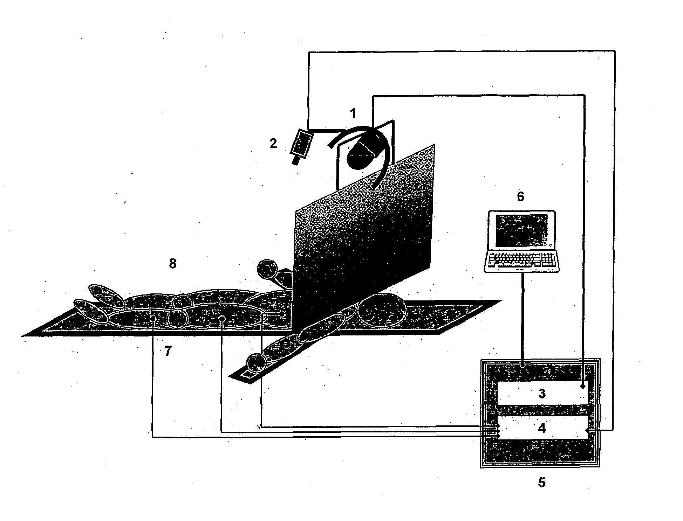

Eine Ausführungsform der Erfindung wird im folgenden anhand der Zeichnung beschrieben. Ein schwenkbarer Infrarotstrahler 1 mit gerichteter Abstrahlcharakteristik ist oberhalb eines Operationstisches angeordnet. Ihm ist ein schwenkbares Pyrometer 2 zur kontaktlosen Temperaturmessung unterschiedlicher Oberflächenpartien zugeordnet. Eine mit einem Computer 6 verbundene Regelungseinheit 5 steuert den schwenkbaren Infrarotstrahler 1 an und empfängt Regelsignale von dem schwenkbaren Pyrometer 2. Die Leistungssteuerung in der Regelungseinheit 5 ist bei 3 angedeutet. Eine Temperaturerfassungseinheit 4 empfängt die bereits erwähnten Signale von dem Pyrometer 2 sowie von im vorliegenden Ausführungsbeispiel 3 zusätzlichen Kontakttermometern mit der Bezugsziffer 7, die die Haut- und Kerntemperatur des Patienten 8 erfassen.

## Patentansprüche

1. Infrarotbestrahlungseinrichtung zur Bestrahlung der menschlichen Haut einer Person, mit einer Infrarotquelle und einer Leistungsregeleinrichtung (5) für die Infrarotquelle, **dadurch gekennzeichnet, dass** zur Leistungsregelung der Infrarotquelle ein Regelkreis vorgesehen ist, in den als Regelgrößen sowohl die Temperatur wenigstens eines Teils der bestrahlten Hautfläche der Person als auch die Kerntemperatur der Person einfließen.

2. Infrarotbestrahlungseinrichtung nach Anspruch 1, **dadurch gekennzeichnet, dass** Strahlungssensoren (2) (Pyrometer) und/oder Hautkontakthermometer (7) für die Hauttemperaturmessung vorgesehen sind.

3. Infrarotbestrahlungseinrichtung nach Anspruch 1 oder 2, **dadurch gekennzeichnet, dass** Kontaktthermometer (7) für die Messung der Kerntemperatur vorgesehen sind.

4. Infrarotbestrahlungseinrichtung nach einem der Ansprüche 1 bis 3, **dadurch gekennzeichnet, dass** die Strahlungsleistung 1.000 W/m² bestrahlte Hautfläche oder weniger, vorzugsweise 500 W/m² bestrahlte Hautfläche oder weniger beträgt.

5. Infrarotbestrahlungseinrichtung nach einem der Ansprüche 1 bis 4, **dadurch gekennzeichnet, dass** die Infrarotstrahlung auf einstellbare Flächenbereiche fokussierbar ist.

6. Infrarotbestrahlungseinrichtung nach einem der Ansprüche 1 bis 5, **dadurch gekennzeichnet, dass** sie einen Sensor zur Messung der IR-Reflexivität der bestrahlten Flächen aufweist.

7. Infrarotbestrahlungseinrichtung nach Anspruch 6, **dadurch gekennzeichnet, dass** sie eine Einrichtung zur Umrisserkennung der zu bestrahlenden Hautflächen einer Person durch Messung der IR-Reflexivität der bestrahlten Flächen aufweist.

8. Infrarotbestrahlungseinrichtung nach einem der Ansprüche 1 bis 6, **dadurch gekennzeichnet, dass** sie eine Einrichtung zur Umrisserkennung der zu bestrahlenden Hautflächen einer Person durch Messung der Reflektionen im sichtbaren Bereich aufweist.
